# EUROPEAN PATENT APPLICATION

(11) **EP 0 799 637 A1**
(43) Date of publication of application: **08.10.1997**
(21) Application number: 97301715.5
(22) Date of filing: 13.03.1997
(51) Int. Cl.: B01J 3/00, B01J 3/04, A61L 2/24

(54) **Safety system for autoclave apparatus**

(30) Priority: 03.04.1996 GB 9607070
(71) Applicant: Smiths Industries Public Limited Company, London, NW11 8DS (GB)
(72) Inventor: Brake, Stephen John, Lancing, West Sussex BN15 9QX (GB); McBrien, Peter Anthony, Worthing, West Sussex BN14 9RJ (GB)
(74) Representative: Flint, Jonathan McNeill

(57) **Abstract**

An autoclave has a pressure sensor 20 and a door sensor 6. When pressure in the chamber 2 rises before the door 5 has been fully closed, the autoclave turns on a pump 12 to reduce pressure in the chamber. When pressure has fallen below atmosphere, a valve 17 is opened to allow air to enter the chamber through a filter 16 and equalize pressure inside and outside the chamber.

## Description

This invention relates to autoclave apparatus of the kind including pressure chamber with a door, a door sensor that provides an output signal indicating that the door is not fully closed, a pressure sensor responsive to pressure in the chamber, a pump, a filtered inlet and a valve operable to prevent or enable flow of gas into the chamber via the inlet.

Autoclaves have a pressure chamber in which an article to be treated is placed, the chamber being closed by a door so that the article can be subject to steam or other vapour at elevated temperature and pressure. In a busy dental practice, for example, the sterilizer may need to be reused very soon after the end of a sterilization cycle. There is, therefore, considerable stored heat in the shell of the sterilizer when the door is closed to start the next cycle. This can create a problem, because the stored heat rapidly warms the air in the chamber and, as soon as the door is pushed closed, pressure starts to build up in the chamber. The amount and speed of this increase in pressure can be sufficient to prevent the door being fully closed or being reopened. This is a particular problem in autoclaves where the door is locked by rotation. It is a particular problem in autoclaves having a vacuum pump for producing a vacuum cycle because they do not have a constant air bleed. which would prevent the pressure rise. When the door is jammed closed in this way, the user has to wait for the apparatus to cool sufficiently for the pressure to drop closer to atmosphere in order to close or open the door.

It is an object of the present invention to provide an improved autoclave apparatus.

According to one aspect of the present invention there is provided autoclave apparatus of the above-specified kind, characterised in that the apparatus is arranged such that, when the door sensor and pressure sensor indicate excess pressure in the chamber and incomplete closure of the door, the pump is operated to reduce the pressure in the chamber below atmosphere, and that the apparatus is arranged subsequently to open the valve to allow air to flow into the chamber via the filter to equalize pressure inside and outside the chamber.

The valve is preferably a solenoid valve. The apparatus may include a manual switch, which, when operated, turns on the vacuum pump and then causes the valve to open.

According to another aspect of the present invention there is provided a method of reducing pressure in an autoclave chamber following an increase in pressure above atmosphere while the door to the chamber is incompletely closed, comprising the steps of pumping down the chamber to a pressure below atmosphere and subsequently allowing filtered air into the chamber to equalize pressure in the chamber with atmosphere.

According to a further aspect of the present invention there is provided a method of operating an autoclave comprising the steps of detecting an increase in pressure within the autoclave chamber when the door of the chamber is not fully closed, pumping down the chamber to a pressure below atmosphere and subsequently allowing filtered air into the chamber to equalize pressure in the chamber with atmosphere.

According to yet another aspect of the present invention there is provided a method of operating an autoclave comprising the steps of monitoring the output of a pressure sensor responsive to pressure in the autoclave chamber, monitoring the output of a door sensor responsive to closure of the door, detecting when the pressure sensor output and the door sensor output indicate an elevated pressure in the chamber and incomplete closure of the door, turning on a pump to reduce the pressure in the chamber below atmosphere when there is an elevated pressure in the chamber and incomplete closure of the door and subsequently opening a valve to allow air to flow back into the chamber via a filter so as to allow pressure in the chamber to equalize with atmosphere.

The method may include the step of fully closing the door after pressure in the chamber has equalized and restarting the autoclave treatment cycle.

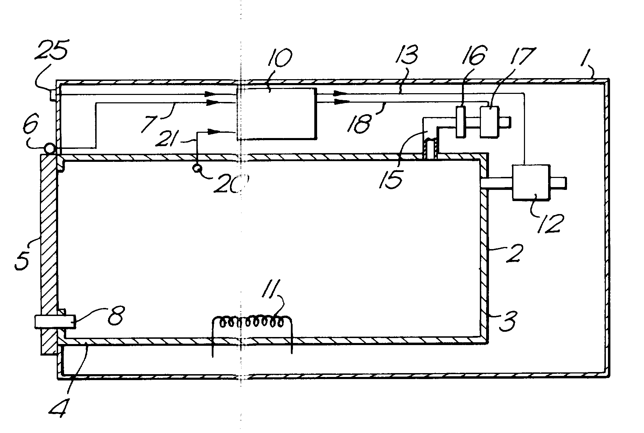

An autoclave sterilizer and its method of operation, in accordance with the present invention, will now be described, by way of example, with reference to the accompanying drawing, which is a cross-sectional side elevation of the sterilizer.

The sterilizer has an outer housing 1 containing a cylindrical pressure chamber 2. The rear end 3 of the chamber is closed and the front end 4 is open but can be closed by a conventional door 5. The door 5 is hinged along one side about a vertical axis and is fully closed by rotating it through an angle of about 45 degrees once it has been pushed into position against the front of the chamber. A sensor 6 mounted adjacent the door 5 detects its position, that is, whether or not it is fully closed. The sensor 6 provides an output via line 7 to a control unit 10. A pressure-responsive safety lock 8 engages the door when triggered by elevated pressure in the chamber 2, to prevent the door being opened.

A heating element 11 in the chamber 2 is submerged in water at the start of a sterilization cycle so as to produce steam within the chamber, in the usual way. The sterilizer also has a vacuum pump 12 with an inlet opening into the chamber 2 towards its upper end. The pump 12 is controlled by signals along line 13 from the control unit 10, to pump out air and vapour from the chamber 2 at different phases of the sterilization cycle.

The chamber 2 has a vent opeining 15 at its upper end, which communicates via a filter 16 with a solenoid-operated valve 17. The valve 17 is normally held closed so that no gas can escape from or enter the chamber 2 through the opening 15. The valve can be opened by signals from the control unit 10 via line 18.

A pressure sensor 20 in the chamber 2 provides a pressure signal via line 21 to the control unit 10.

The sterilizer has various other conventional features but it is not necessary to describe these to understand fully the present invention.

In normal operation, the sterilizer functions in the usual way. If, however, the sterilizer is reused shortly after the end of a sterilization cycle, there is a risk that residual heat in the wall of the chamber 2 could cause a build-up of pressure immediately following the door 5 being pushed partially closed. This rise in pressure may be so quick that it causes the safety lock 8 to trigger before the door 5 has been fully closed. This does not present any danger, because the door sensor 6 indicates incomplete closure of the door 5, thereby preventing the sterilization cycle being started. However, it can prevent the door 5 being fully closed or opened until pressure in the chamber 2 has fallen. The control unit 10 detects the nature of the fault because it receives an elevated pressure signal from the pressure sensor 20 and an incomplete door closure signal from the door sensor 6. In response to this, the control unit 10 turns on the vacuum pump 12 until pressure in the chamber falls below atmosphere. as indicated by the output from the pressure sensor 20. The control unit 10 then turns off the vacuum pump 12 and opens the valve 17 so that air can flow into the chamber 2 via the filter 16, allowing the pressure in the chamber to equalize with atmosphere. The safety lock 8 then releases, allowing the door 5 to be fully closed and the sterilization treatment cycle restarted. The sterilizer also has, on its front panel, a manual switch 25 connected to the control unit 10 so that the user can manually start the pressure equalization procedure if he wishes. If desired. this manual switch 25 can replace the automatic pressure equalization procedure in some sterilizers.

Because pressure in the chamber 2 is first reduced below atmosphere. the present invention ensures that the chamber-side of the filter 16 is not contaminated by unfiltered or unsterilized air. By contrast, if the vacuum phase were omitted and excess pressure were simply discharged to atmosphere via the filtered vent 15, the chamber-side of the filter 16 could become contaminated by the escaping air because this is untreated, non-sterile air. Subsequent operation of the sterilizer could result in contamination of articles in the chamber 2 when air is admitted through the contaminated filter. The present invention, therefore, enables rapid release of a jammed door without the risk of contamination.

## Claims

1. Autoclave apparatus including a pressure chamber with a door, a door sensor that provides an output signal indicating that the door is not fully closed, a pressure sensor responsive to pressure in the chamber, a pump, a filtered inlet and a valve operable to prevent or enable flow of into the chamber via the inlet. characterised in that the apparatus is arranged such that, when the door sensor (6) and pressure sensor (20) indicate excess pressure in the chamber (2) and incomplete closure of the door (5), the pump (12) is operated to reduce the pressure in the chamber below atmosphere, and that the apparatus is arranged subsequently to open the valve (17) to allow air to flow into the chamber via the filter (16) to equalize pressure inside and outside the chamber.

2. Autoclave apparatus according to Claim 1, characterised in that the valve is a solenoid valve (17).

3. Autoclave apparatus according to Claim 1 or 2, including a manual switch (25), which, when operated, turns on the vacuum pump (12) and then causes the valve (17) to open.

4. A method of reducing pressure in an autoclave chamber following an increase in pressure above atmosphere while the door (5) to the chamber (2) is incompletely closed, comprising the steps of pumping down the chamber to a pressure below atmosphere and subsequently allowing filtered air into the chamber to equalize pressure in the chamber with atmosphere.

5. A method of operating an autoclave comprising the steps of detecting an increase in pressure within the autoclave chamber when the door (5) of the chamber (2) is not fully closed, pumping down the chamber to a pressure below atmosphere and subsequently allowing filtered air into the chamber to equalize pressure in the chamber with atmosphere.

6. A method of operating an autoclave comprising the steps of monitoring the output of a pressure sensor (20) responsive to pressure in the autoclave chamber (2), monitoring the output of a door sensor (6) responsive to closure of the door (5), detecting when the pressure sensor output and the door sensor output indicate an elevated pressure in the chamber and incomplete closure of the door, turning on a pump (12) to reduce the pressure in the chamber below atmosphere when there is an elevated pressure in the chamber and incomplete closure of the door, and subsequently opening a valve (17) to allow air to flow back into the chamber via a filter (16) so as to allow pressure in the chamber to equalize with atmosphere.

7. A method according to Claim 5 or 6 including the step of fully closing the door (5) after pressure in the chamber (2) has equalized and restarting the autoclave treatment cycle.
